Europäisches Patentamt

European Patent Office

Office européen des brevets

(11) Numéro de publication: **0 209 451**
**A1**

(12) **DEMANDE DE BREVET EUROPEEN**

(21) Numéro de dépôt: 86401517.7

(22) Date de dépôt: 08.07.86

(51) Int. Cl.4: **C12Q 1/68** , C07H 21/04 , C12N 15/00 , //C12N9/16

(30) Priorité: 08.07.85 FR 8510454

(43) Date de publication de la demande:
21.01.87 Bulletin 87/04

(84) Etats contractants désignés:
**AT BE CH DE FR GB IT LI LU NL SE**

(71) Demandeur: **INSTITUT PASTEUR**
**25/28, rue du Docteur Roux**
**F-75015 Paris(FR)**
Demandeur: **CENTRE NATIONAL DE LA**
**RECHERCHE SCIENTIFIQUE (CNRS)**
**15, Quai Anatole France**
**F-75007 Paris(FR)**

(72) Inventeur: **Andremont, Antoine**
**252, rue de Vaugirard**
**F-75015 Paris(FR)**
Inventeur: **Ounissi, Houria**
**10, avenue St-Rémy**
**F-93200 Saint-Denis(FR)**
Inventeur: **Courvalin, Patrice**
**13, rue Emile Duclaux**
**F-75015 Paris(FR)**

(74) Mandataire: Peaucelle, Chantal et al
S.C. Ernest Gutmann - Yves Plasseraud 67,
boulevard Haussmann
F-75008 Paris(FR)

(54) **Sonde et procédé pour la détection de facteurs de résistance à l'érythromycine dans des cultures cellulaires, notamment bactériennes.**

(57) L'invention concerne un procédé de détection in vitro d'un facteur de résistance à l'érythromycine dans un prélèvement tel qu'une selle humaine. Il comprend la mise en contact des acides nucléiques contenus dans ce prélèvement avec une sonde nucléotidique contenant une partie au moins du gène ereA isolé à partir du plasmide contenu dans la souche de E.coli déposée à la Collection Nationale des Cultures de Microorganismes (CNCM) de l'Institut Pasteur de Paris sous le N° I-454, le 5 Juin 1985.

## Sonde et procédé pour la détection de facteurs de résistance à l'érythromycine dans des cultures cellulaires, notamment bactériennes.

L'invention concerne un acide nucléique, plus particulièrement une sonde et un procédé pour la détection de facteurs de résistance à l'érythromycine dans des cultures cellulaires, notamment bactériennes.

Le problème de la détection d'une résistance bactérienne aux antibiotiques est bien connu. Il se pose de plus en plus en clinique humaine, en raison des résistances acquises par les bactéries en nombre de plus en plus important contre des antibiotiques déterminés. La conséquence en est la nécessité de subordonner dans la mesure du possible toute thérapie avec un antibiotique déterminé à un test de détection préalable de l'existence chez la bactérie infectant le patient à traiter d'une résistance éventuelle à cet antibiotique. Dans le cas d'une réponse positive dans le test en question, l'antibiotique concerné doit être écarté de toute prescription clinique.

L'érythromycine constitue un antibiotique dont a récemment été reconnue l'efficacité pour la prévention et le traitement de certaines infections dues à des microorganismes à Gram-négatif - (Andremont A. et al : Ann. Inst. Pasteur, Paris, 132 B (1981) 419-427 et Andremont A. et al, J. Infect. Dis. 148 (1983) 579-587). Les infections concernées intéressent souvent la sphère gastro-intestinale et se manifestent par des diarrhées importantes. L'agent infectieux peut alors être isolé, par exemple à partir des selles des patients affectés par de telles infections.

Le dépistage de l'existence d'une résistance possible à l'érythromycine peut, comme il est courant pour d'autres antibiotiques, être étudié par des méthodes classiques, mettant en jeu la détection de la conséquence phénotypique qu'elle entraîne sur la bactérie pathogène, tant en bactériostase qu'en bactéricidie, après mise en culture de l'échantillon biologique susceptible de la contenir, dans l'exemple précédent d'un prélèvement de selle diarrhéique. Ces techniques, pour efficaces qu'elles soient, présentent un inconvénient majeur, celui de la durée nécessaire pour la réalisation des cultures préalables de la flore bactérienne contenue dans le prélèvement initial, et de l'isolement de l'agent pathogène pour réaliser la détection phénotype adéquate. Cette durée peut alors être incompatible avec la nécessité d'intervenir rapidement dans le cas d'une urgence thérapeutique.

L'utilisation de méthodes d'hybridation entre la séquence génique responsable d'une résistance à un antibiotique et contenue dans l'agent pathogène responsable avec une sonde appropriée n'a guère été envisagée jusqu'à ce jour, bien qu'a priori cette technique présente les avantages suivants :

-la sensibilité, avec pour corollaire une diminution, voire la suppression, du temps de culture bactérienne et la possibilité de travailler directement sur le produit pathologique (prélèvement),

-la transposition des résultats du phénotype au génotype avec pour conséquence la suppression de l'identification bactérienne (agent pathogène).

Cependant, la banalisation de cette technique est retardée du fait :

-de la difficulté de travail de certains produits pathologiques, puis notamment,

-de l'aspect paucimicrobien de certains prélèvements, notamment dans le cas de maladies sexuellement transmissibles,

-enfin et surtout, de l'ignorance dans laquelle on se trouve encore de l'existence d'une éventuelle hétérogénéité génique pour un caractère de résistance donné.

C'est ainsi qu'avait déjà été reconnue l'hétérogénéité des déterminants de la résistance à la tétracycline (Mendez B. et al.,(1980) Plasmid 9, 99-108) et celles des déterminants de la résistance à des antibiotiques du type macrolide - lincosamide-streptogramine-B (Ounissi et al. (1982) , Microbiology-1982, édité par D. Schlessinger, American Society for Microbiology (Société Américaine de Microbiologie), p. 167-169).

L'invention découle de la découverte que les facteurs de résistance à l'érythromycine pouvaient au contraire être détectés avec un grand degré de certitude, en mettant en oeuvre dans des essais d'hybridation une sonde nucléotidique contenant une partie au moins du gène ereA codant pour une estérase capable d'hydrolyser le cycle lactone de l'érythromycine. La séquence nucléotidique - (contenant 1032 paires de bases) du gène ereA est la suivante :

ATGC GATTGGTTTG GAAATGTGGG GCGATT-
CAGG CATCCCGGTT ATCTGAATGG CTCAACT-
CAA CAGCCGGTGC TCATGAACTT GAGC-
GATTTT CGGATACCCT GACCTTTTCT GTG-
TATGGCT CAGTGCTGAT CTGGCTGAAA TCA-
TATCTCC GCGAATCAGG AAGAAAACTG CAGT-
TAGTCG GAATCGCCTT ACCCAACACC CT-
GAACCCAA GGGACGACCT AGCGCAATTG GCC-
GAAATTA TCCAGCTCAT CGATCACCTC AT-
GAAACCGC ACGTTGATAT GTTGACTCAC

TTGTTGGCGT CCATTGATGG CCAGTCGGCG
GTTATTTCAT CGGCAAAATG GGGGGAGCTA
GAAACGGCTC GGCAGGAGAA AGCTATCTCA
GGGGTAACCA GATTGAAGCT CCGCTTGGCG
TCGCTTGCCC CCGTCCTGAA AAAACACGTC AA-
CAGCGATT TGTTCCGAAA AGCCTCTGAT CGAA-
TAGAGT CGATAGAGTA TACGTTGGAA
ACCTTGCGTA TAATGAAAAC TTTCTTCGAT GG-
TACCTCTC TTGAGGGAGA TACTTCCGTA CGT-
GACTCGT ATATGGCGGG CGTAGTAGAT
GGAATGGTTC GAGCGAATCC GGATGTGAAG
ATAATTCTGC TGGCGCACAA CAATCATCTA
CAAAAAACTC CAGTCTCCTT TTCAGGCGAG CT-
TACGGCTG TTCCCATGGG GCAGCACCTC GCA-
GAGAGGG TGAATTACCG TGCGATTGCA TT-
CACCCATC TTGGACCCAC CGTGCCGGAA ATG-
CATTTCC CATCGCCAAA AAGTCCTCTT
GGATTCTCTG TTGTGACCAC GCCTGCCGAT
GCAATCCGTG AGGATAGTAT GGAACAGTAT
GTCATCGACG CCTGTGGTAC GGAGAATTCA
TGTCTGACAT TGACAGATGC CCCCATGGAA
GCAAAGCGAA TGCGGTCTCA AAGCGCCTCT
GTAGAAACGA AATTGAGCGA GGCATTTGAT
GCCATCGTCT GTGTTACAAG CGCCGGCAAG
GACAGCCTGG TTGCCCTA

Cette séquence (qui comporte une phase ouverte de lecture de 1032 paires de bases) a été isolée à partir d'un plasmide pIP1100 de 61 kb, dont a été reconnue la capacité de conférer une forte résistance à <u>Escherichia coli</u> contre l'érythromycine. La masse moléculaire de la chaîne polypeptidique de l'estérase de l'érythromycine, telle qu'elle peut être déduite de la séquence nucléotidique du gène <u>ereA</u> est de 37765, une valeur en accord avec celle déterminée expérimentalement (environ 43000) par électrophorèse en gel de dodecyl sulfate de sodium-polyacrylamide (SDS-PAGE). Ce plasmide a été lui-même isolé d'une souche de <u>E. coli</u> isolée au laboratoire à partir d'un donneur humain et présentant un haut degré de résistance à l'érythromycine (à des doses supérieures à 2 mg/ml) ainsi qu'à d'autres antibiotiques, tels que l'ampicilline, la gentamicine et la streptomycine.

La reproductibilité de la détection a été étudiée en utilisant une sonde intragénique contenant une insertion de 720 paires de bases prélevé entre les sites <u>EcoRI-PstI</u> du gène <u>ereA</u>, et en réalisant des essais d'hybridation sur des colonies en grand nombre, provenant de bactéries pathogènes appartenant à des genres différents. L'extrême stabilité du gène doit résulter de la stabilité du plasmide pIP1100 dans son hôte initial, la souche <u>E. coli</u> BM 2195 à partir de laquelle il a été isolé, et de sa capacité à être transféré avec une fréquence élevée, $10^{-4}$, à d'autres cellules d' <u>E. coli</u>, dans

lesquelles il reste très stable. Ce comportement suggère que les appareils de réplication et de transfert du plasmide pIP1100 particulièrement bien appropriés à <u>E. coli</u>.

Les conditions dans lesquelles le gène <u>ereA</u> a été isolé et séquencé sont décrites plus loin.

Le procédé selon l'invention de détection de l'existence d'un facteur de résistance à l'érythromycine dans un échantillon biologique, notamment de selles, est donc caractérisé par la mise en contact de cet échantillon ou des acides nucléiques que celui-ci contient avec une sonde contenant une séquence nucléotidique contenant une partie au moins du gène <u>ereA</u> dans des conditions autorisant l'hybridation éventuelle de cette séquence avec les acides nucléiques codant pour le facteur de résistance correspondant éventuellement contenu dans l'échantillon.

Il va naturellement de soi que l'expression "partie au moins de gène <u>ereA</u>", telle qu'elle est définie dans ce qui précède, s'entend comme signifiant également toute séquence nucléotidique plus courte contenue à l'intérieur de ce gène et présentant une longueur suffisante pour donner une réponse caractéristique et non équivoque quant à la spécificité de la capacité de reconnaissance de la présence du gène dans un prélèvement à l'étude, dans des essais d'hybridation mettant en jeu une sonde contenant ladite séquence nucléotidique. Le caractère de spécificité est notamment acquis, dès lors que ladite séquence nucléotidique contient par exemple au moins 15, de préférence 50 nucléotides dans un ordre identique à celui qui prévaut à l'intérieur de l'une des séquences contenues à l'intérieur du gène <u>ereA</u>. L'expression "partie au moins de gène <u>ereA</u>" s'étend également à toute sonde contenant une séquence nucléotidique hybridable avec la précédente, telle qu'obtenue par transcription enzymatique inverse de l'ARN correspondant ou encore par synthèse chimique.

L'invention concerne encore en tant que produit nouveau une séquence nucléotidique d'au plus 1500 paires de bases, telle qu'isolée à partir du plasmide de pIP1100, notamment par clivage à l'aide d'enzymes de restriction appropriées, cette séquence contenant tout ou partie du gène <u>ereA</u> lui-même. L'invention concerne également encore en tant que produit nouveau tout ADN recombinant formé entre la séquence nucléotidique précédente et un acide nucléique étranger au plasmide pIP1100 En particulier, l'invention concerne des vecteurs du type phage, cosmide ou plasmide, modifiés par une partie de gène <u>ereA</u> et permettant sa réplication et son amplification aisées, plus particulièrement dans <u>E. coli</u>.

L'invention concerne enfin les sondes marquées, telles qu'elles peuvent être produites à partir des ADNs précédemment définis, recombinés ou non, notamment des sondes marquées par un élément radioactif ou par tout autre groupe permettant le couplage ultérieur de la sonde, avec une molécule ou un groupe permettant sa reconnaissance à l'état hybridé avec la préparation d'ADN étudiée, par exemple une enzyme dont peut être reconnue l'action sur un substrat ou un produit fluorescent. Des caractéristiques supplémentaires de l'invention apparaîtront encore au cours de la description qui suit des conditions dans lesquelles le gène ereA a été identifié et isolé et dans lesquelles il peut être mis en oeuvre dans des essais d'hybridation. Il sera également fait référence dans ce qui suit aux dessins dans lesquels :

-la figure 1 est une carte de restriction d'une insertion de 1,66 kb issu du plasmide pIP1100 , inséré dans un autre plasmide et contenant le gène ereA ;

-la figure 2 est représentative de la séquence d'un acide nucléique contenu dans l'insertion susdite et montrant dans un encadrement la séquence du gène ereA entier ;

-la figure 3 est illustrative de la stratégie qui a été adoptée pour séquencer l'insertion de 1,66 kb dont question dans la figure 1 ;

-la figure 4 est un autoradiogramme de polypeptides (parmi lesquels l'estérase de l'érythromycine) marqués par la $[^{35}S]$L-méthionine obtenus dans un essai de séparation par SDS-PAGE.

L'attention du lecteur est encore attirée sur la description plus complète des figures telle qu'elle apparaît à la fin de cette description.

On indique ci-après les natures des matériels et des méthodes qui ont été utilisés pour identifier et isoler le gène ereA.

## MATERIAUX ET METHODES

### (a) Souches de bactéries

Les sources et les propriétés des souches bactériennes sont énumérées dans le tableau 1 suivant :

### TABLEAU I

| Souche | Génotype important |
|---|---|
| E. coli K12C600 | thr, leu, thi, lacY |
| | tonA, strA |
| E. coli BM694 | prototrophe, gyrA |
| E. coli K12JM101 | Δ(lac, pro), thi, supE, |
| | F'(traD36, proAB, lacI$^q$, |
| E. coli K12AR1062 | thr, leu, lac, gal, xyl, mal, mtl, hsdS |

Les fragments d'ADN à séquencer sont transfectés dans E. coli JM 101 (1) en utilisant soit le bactériophage M13mp 8, soit M13mp 9 (2).

### (b) Milieux

On utilise de l'agar (Difco) et un bouillon d'une infusion cerveau-coeur. Les tests de sensibilité sur disques sont effectués sur de l'agar Mueller-Hinton. Toutes les incubations sont effectuées à 37°C.

### (c) Plasmides

Les plasmides utilisés dans ces travaux sont énumérés dans le tableau II.

TABLEAU II

| N° du plasmide | Caractères phénotypiques du plasmide (a) | Origine |
|---|---|---|
| pIP1100 | Tra+, IncX, Ap Em Cm Sm | plasmide naturel |
| pAT61 | Tra-, Mob-, Ap Em Sm Tc | pBR322Ω(pIP1100 AvaI(ereA aadA)-3.5 kb) |
| pAT63 | Tra-, Mob-, Ap Em | pBR322Ω(pAT61 partial Sau3a(ereA)-1.66 kb) |
| pAT64 | Tra-, Mob-, Cm Em | pBR329Δ(PstI)Ω (pAT63 HindIII-BamHI(ereA)-2 kb) |
| pAT65 | Tra-, Mob-, Cm | pAT64Ω(λ PstI - 164 bp) |
| pBR329Δ(PstI) | Tra-, Mob-, Cm Tc | construction in vitro |
| pUC8 | Tra-, Mob-, Ap | construction in vitro |

(a) Nomenclature des caractères phénotypiques des plasmides selon (12).

Le site PstI dans pBR329 est éliminé comme suit. L'ADN plasmidique de pBR329 est linéarisé avec PstI introduit dans E. coli par transformation avec sélection pour la résistance à la tétracycline et au chloramphénicol, mais non à l'ampicilline. Un plasmide résistant au clivage avec PstI est sélectionné et appelé pBR329Δ(PstI).

(d) Préparation d'ADN

L'isolement de l'ADN plasmidique pIP 1100 et l'isolement à grande échelle de pBR322 et de l'ADN de plasmides dérivés de ce dernier sont effectués comme décrit dans (3) et (4).

(e) Electrophorèse et purification de fragments d'ADN générés après digestion par des endonucléases de restriction

Les fragments d'ADN restreints sont séparés par électrophorèse dans des gels sur plaques horizontales (20x20x0,7cm) contenant 0,8 % d'agarose se gélifiant à basse température ou dans des gels sur plaques verticales (20x40x0,3cm) contenant 5 % d'acrylamide et 0,25 % de bis-acrylamide. Les fragments d'ADN sont purifiés comme décrit par Maniatis et al. (1982) dans (5).

(f) Séquençage d'ADN

Les fragments d'ADN sont clonés dans les bactériophages M13mp8 et M13mp9 et séquencés par la technique de terminaison de chaînes selon Sanger et al.,(6). La séquence d'ADN complète est agencée en utilisant les programmes d'ordinateurs DBCOMP et DBUTIL (Staden) (7).

(g) Analyse des minicellules

Les plasmides sont introduits par transformation selon Davis et al . (4) dans la souche AR1062 produisant des minicellules et les minicellules sont préparées comme décrit par Rambach et Hogness (Proc.Natl. Acad. Sci. USA 74 (1977) 5051.5045). Le marquage des polypeptides avec de la [³⁵S] - méthionine et l'électrophorèse sur polyacrylamide contenant du dodécyl-sulfate de sodium sont effectués comme décrit par Laemmli U.K. (Nature 227, 680-685 (1970).

(h) Enzymes

En suivant les recommandations des fabricants (Boehringer Mannheim), on utilise les endonucléases de restriction (BamHI, EcoRI, HpaII, HindIII, PstI et Sau3A), l'ADN polymérase I, - (fragment large) , l'ADN ligase du bactériophage T4, la phosphatase alcaline de veau. On utilise le lysozome de Sigma.

(i) Produits chimiques

On utilise le triphosphate de déoxyadénosine 5'-[$\alpha^{32P}$] le sel de triéthylammonium et la L-méthionine [$^{35}S$] obtenus de Radiochemical Center Amersham. Les triphosphates de désoxynucléosides , les triphosphates de didésooxynucléosides et les ADN des bactériophages M13mp8 et M13mp9RF proviennent de PL-Biochemicals. L'ADN du Bactério phage λ cl 857 Sam7 DNA provient de Boehringer. M13 pentadécamère provient de Biolabs. Les marqueurs de poids moléculaire pour SDS-PAGE sont de LKB. Les antibiotiques proviennent des Laboratoires suivants :

-ampicilline (Bristol),

-chloramphénicol et érythromycine (Roussel-Uclaf),

-streptomycine et tétracycline (Pfizer).

EXEMPLE 1 :

a) Clonage moléculaire du déterminant de résistance à l'érythromycine du plasmide pIP1100-

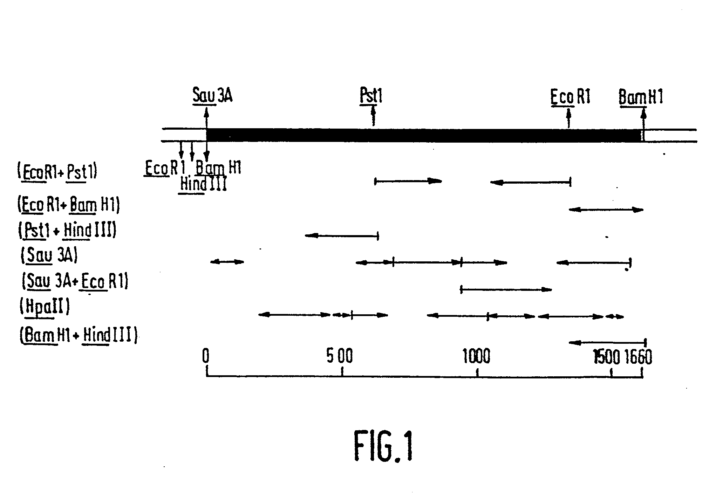

On mélange de l'ADN de plasmide pIP 1100 - (Tra⁺, IncX, Ap Em Gm Sm, 61 kb) et de plasmide pBR322 (Ap Tc, 4.36 kb), on digère à l'aide de l'endonucléase Aval, on ligue on introduit dans E. coli par transformation et les clones sont sélectionnés sur de l'érythromycine (200 μg/ml). Le contenu plasmidique des transformants qui sont résistants à l'ampicilline et à l'érythromycine mais également à la streptomycine est analysée par électrophorèse sur gel d'agarose des lysats bactériens bruts selon (8). L'ADN plasmidique de l'un des transformants ayant le plus petit hybride est digéré par Aval et l'insertion de 3,5 kb de pIP1100 est purifié et digéré partiellement avec Sau3A. Les fragments de restriction obtenus sont clonés dans pBR322, digérés avec BamHI et les transformants sont sélectionnés sur érythromycine, puis répliqués sur streptomycine. La teneur en plasmide des transformants résistants à l'ampicilline et à l'érythromycine seulement est analysée par électrophorèse sur gel d'agarose. L'ADN du plus petit hybride, pAT63 est purifié et analysé par électrophorèse sur gel d'agarose après digestion avec les endonucléases Sau3A, EcoRI, Pst1 ou Hind III plus BamHI. Le plasmide pAT63 est constitué par pBR322 plus une insertion de 1,66 kb d'ADN de pIP 1100 (figure 1, tableau II).

Les fragments d'ADN de pAT63 digérés par EcoRI-HindIII (1750 pb) et BamHI-PstI (970 pb) sont sous-clonés dans pUC8. Ces fragments ne confèrent pas de résistance à l'érythromycine.

b) Inactivation par insertion in vitro du gène ereA.

L'ADN du plasmide pAT63 est digéré avec HindIII plus BamHI. Le fragment d'ADN 2 kb qui contient l'insérat entier Sau3A (figure 1), est purifié et cloné dans pBR329 Δ (PstI) (tableau II) digéré à l'aide de HindIII plus BamHI. Les transformants sont sélectionnés sur leur résistance à l'érythromycine et au chloramphénicol mais non à la tétracycline. Le plasmide pAT64 de l'un des transformants est purifié, digéré avec PstI, déphosphorylé, mélangé avec l'ADN purifié du fragment de 164 pb PstI du bactériophage λ (9) soumis à une étape de ligation, introduit dans E. coli par transformation et les clones sont sélectionnés pour leur résistance au chloramphénicol mais non à l'érythromycine. L'ADN plasmidique d'un transformant est purifié et analysé par électrophorèse sur gel de polyacrylamide après digestion avec l'endonucléase PstI. Le plasmide pAT65 est constitué de pAT64 plus l'insertion d'ADN λ de 164 pb.

c) Analyse de l'estérase d'érythromycine dans des minicellules.

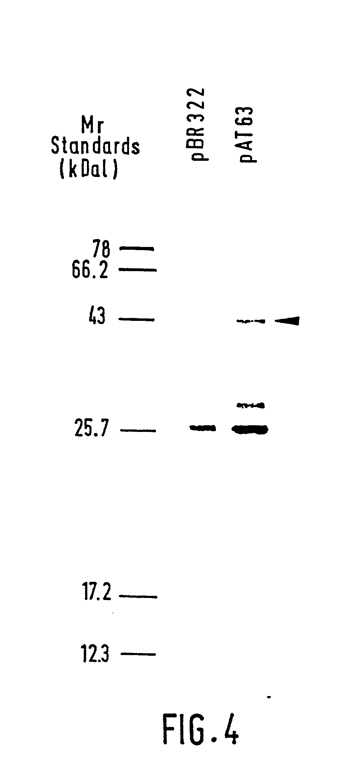

Les plasmides pBR322 et pAT63 sont introduits dans la souche d'E. coli AR1062 qui produit des minicellules. Après purification des minicellules et marquage des polypeptides, les protéines sont analysées par SDS-PAGE. On observe une bande, qui doit correspondre à l'estérase d'érythromycine avec une masse moléculaire estimé d'approximativement 43000, avec pAT63 mais non avec pBR322 (voir figure 4).

d) Séquence nucléotidique de l'insertion dans pAT63

On digère l'ADN purifié du fragment de 2 kb HindIII-BamHI du plasmide pAT63 (figure 1) indépendamment avec diverses endonucléases de restriction qui permettent ensuite le clonage moléculaire dans les formes réplicatives des bactériophages M13mp8 ou M13mp9. Dans chaque expérience les insertions des clones sélectionnés au hasard sont séquencés par la technique de terminaison de chaîne. La stratégie de séquençage est représentée sur la figure 1. Toutes les séquences sont déterminées sur les deux brins d'ADN obtenus à partir des clones ayant des insertions se recoupant et/ou à partir des clones ayant la même insertion cloné dans les deux orientations.

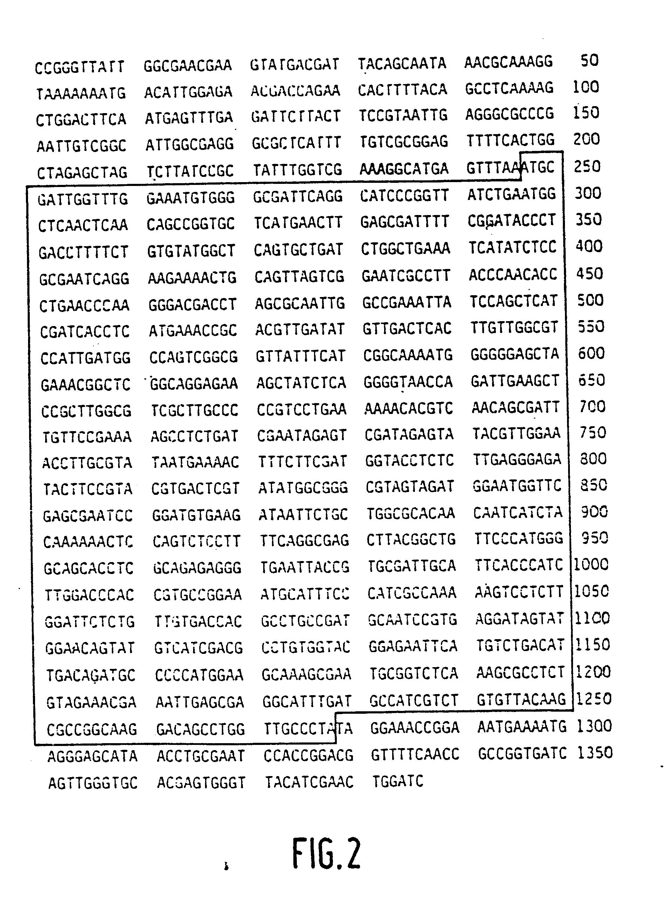

La séquence nucléotidique entière obtenue par analyse à l'ordinateur est représentée sur la figure 2. Elle est constituée par 1386 paires de base, étant donné que la séquence de 150 pb environ distante de 124 pb du site HindIII n'a pas été effectuée (figure 1).

e) Identification du gène codant pour l'érythromycine-estérase.

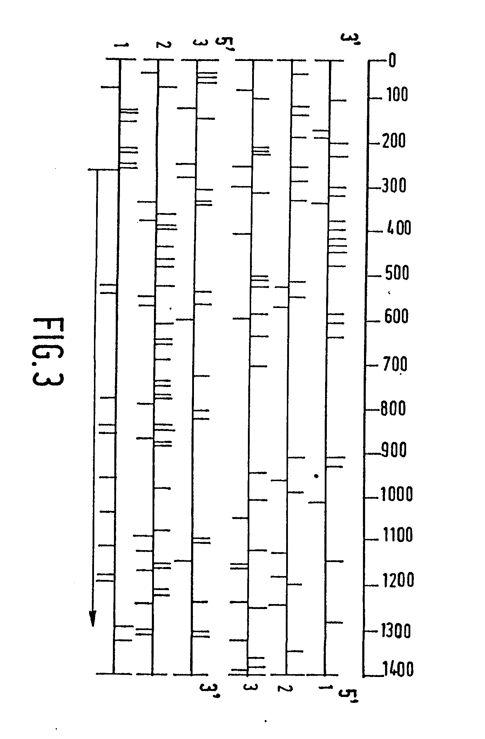

La localisation des codons de terminaison - (TAG, TGA, TAA) dans les trois cadres de lecture de chaque brin d'ADN séquencé (figure 3) indique une seule phase de lecture ouverte pour l'estérase.

Cette région s'étend du codon TAA à la position 244 au codon TAG à la position 1279.

Un test statistique a été utilisé pour vérifier si ce fragment d'ADN correspond à la région codant ou ne codant pas la protéine (10). La probabilité de codage trouvée est de 92 %, ce qui correspond à une prédiction de codage. Le codon ATG à la position 247 est le seul initiateur (ATG, GTG, TTG) précédé par une séquence du type de fixation au ribosome ou SFR dans les 200 premiers nucléotides de cette phase ouverte de lecture. Dix paires de base en amont de ce codon ATG, est localisée une séquence consensus présumée - (AAAGGCAT) SFR (fig. 2)

Cette séquence, faiblement conservée, possède une complémentarité pour seulement 4 des 8 bases (souli gnées) avec le terminaison 3'-OH de l'ARN-r 16S (3'-OH AUUCCUCC 5') de E. coli.

L'énergie libre d'interaction ($\Delta$G) de la structure la plus stable entre la séquence RBS supposée et la terminaison 3'-OH de l'ARN ribosomal 16S, calculée comme décrit par TINOCCO et al. - (11) est -8,4Kcalories mole⁻¹.

Cette valeur est semblable à la valeur moyenne (-9Kcalories mole⁻¹) calculée pour beaucoup de gènes de E . coli.

Les résultats du clonage et la taille estimée pour l'estérase d'érythromycine indiquent que le site PstI (figure 1) est situé dans le gène de structure ereA (figure 2). Ceci est corroboré par le fait que la traduction démarre à partir du codon ATG à la position 247, les autres codons ATG dans la phase étant situés en aval du site PstI.

La séquence nucléotidique qui s'étend de ce codon ATG au codon TAG à la position 1279 peut coder pour une protéine de 344 acides aminés avec une masse moléculaire, calculée sur la base de la composition en aminoacide, de 37 765. Cette valeur est en accord avec celle, 43 000, estimée par électrophorèse sur gel de polyacrylamide SDS.

Dans ce qui suit, on rapporte des commentaires plus détaillés sur les figures 1 à 4 dont question dans l'exemple qui précède.

Sur la figure 1, on a représenté la carte de restriction de l'insertion de 1,66 kb dans le plasmide pAT63. Les segments de vecteur et d'insertion sont représentés respectivement par les segments clairs et foncés.

Le site de restriction de la jonction vecteur-insertion de Sau3A est défini comme position 0. Les fragments d'ADN résultant de la restriction avec les endonucléases indiqués à gauche ont été clonés dans des vecteurs dérivés du bactériophage M13. Les flèches indiquent la direction et l'étendue de la réaction de séquençage. Les tailles sont précisées en pb.

La figure 2 représente la séquence d'ADN du fragment de 1386 pb contenant le gène codant pour l'érythromycine estérase. La numérotation commence au premier site HpaII à gauche de l'insertion du plasmide pAT63. La région codant pour l'érythromycine estérase est encadrée. En lettres plus appuyées, on a indiqué la séquence présumée Shine-Dalgarno.(SFR)

Sur la figure 3, on a représenté les codons d'initiation (ATG) et de terminaison (TAA, TAG, TGA) du fragment de 1386 pb séquencé dans le plasmide pAT63. La numérotation commence au premier site HpaII à gauche dans l'insertion plasmide pAT63. Les codons d'initiation (lignes vers le bas) et de terminaison (lignes vers le haut) sont indiqués pour les trois cadres de lecture des deux brins d'ADN. La flèche indique le gène structural pour l'estérase d'érythromycine.

La figure 4 représente un autoradiogramme de polypeptides marqués avec la L-méthionine [³⁵S] d'une expérience minicellule après séparation par électrophorèse sur gel de polyacrylamide-SDS. La flèche indique l'estérase d'érythromycine.

Les sondes contenant les séquences nucléotidiques selon l'invention peuvent être mises en oeuvre dans des essais de diagnostic in vitro de l'existence ou non dans un prélèvement à étudier d'un facteur de résistance à l'érythromycine, de toute façon en soi connu. On peut par exemple mettre en oeuvre la méthode d'hybridation surcolonies.

Cette méthode comporte, après dénaturation de l'ADN préalablement obtenu à partir de bactéries provenant de selles humaines, le dépôt d'une quantité aliquote de cet ADN sur des membranes de nitrocellulose, l'hybridation de chaque membrane dans les conditions usuelles avec la sonde et la détection de l'hybride formé dans les conditions qui ont été rappelées plus haut.

On peut aussi utiliser une méthode d'hybridation sur réplique. Cette méthode comprend la séparation électrophorétique en gel d'agarose des fragments d'ADNs engendrés après traitement de l'ADN par des enzymes de restriction, le transfert après dénaturation alcaline sur des membranes appropriées et leur hybridation avec la sonde dans les conditions usuelles.

Comme il va de soi et comme il résulte d'ailleurs déjà de ce qui précède, l'invention ne se limite nullement à ceux de ses modes d'application et de réalisation qui ont été plus spécialement envisagés ; elle en embrasse, au contraire, toutes les variantes.

Une culture d'Escherichia coli BM694/pAT63 a été déposée à la Collection Nationale des Cultures de Microorganismes (CNCM) de l'Institut Pasteur de Paris, sous le N° I-454, le 5 Juin 1985.

Les références bibliographiques mentionnées ci-dessous sont les suivantes :

(1) Messing, Tech. Bull 1 (1979) 43-44.

(2) Messing et al., Gene 19 (198 2) 269-276.

(3) Labigne-Roussel et al., Mol. Gen. Genet. 182 (1981) 390-408.

(4) Davis et al., Cold Spring Harbor Lab., Cold Spring Harbor N.Y., 1980

(5) Maniatis et al., Cold Spring Harbor Lab., Cold Spring Harbor, N.Y., 1982, p. 149-186.

(6) Sanger et al., P.N.A.S., USA, 74 (1979), 5463-5467.

(7) Staden Nucl. Acid Res. 8 (1980) 3673-3694.

(8) Birboim et al., Nucl. Acids Res. 7 (1979) 1513-1523.

(9) Sanger et al., J. Mol. Biol. 162 (1982), 729-773.

(10) Fickett, Nucl. Acids Res. 10 (1982) 5303-5318.

(11) Tinocco et al., Nature New Biol. 246 - (1973) 40-41.

(12) Novick et al., Bacteriol. Rev. 40 (1976), 168-169.

**Revendications**

1. Procédé de détection in vitro d'un facteur de résistance à l'érythromycine dans un prélèvement, tel qu'une selle humaine, comprenant la mise en contact des acides nucléiques contenus dans ce prélèvement avec une sonde nucléotidique contenant une partie au moins du gène ereA, dont la séquence est la suivante :

```
ATGC GATTGGTTTG GAAATGTGGG GCGATT-
CAGG CATCCCGGTT ATCTGAATGG CTCAACT-
CAA   CAGCCGGTGC  TCATGAACTT  GAGC-
GATTTT  CGGATACCCT  GACCTTTTCT  GTG-
TATGGCT CAGTGCTGAT CTGGCTGAAA TCA-
TATCTCC GCGAATCAGG AAGAAAACTG CAGT-
TAGTCG  GAATCGCCTT  ACCCAACACC  CT-
GAACCCAA GGGACGACCT AGCGCAATTG GCC-
GAAATTA  TCCAGCTCAT  CGATCACCTC  AT-
GAAACCGC   ACGTTGATAT   GTTGACTCAC
TTGTTGGCGT  CCATTGATGG  CCAGTCGGCG
GTTATTTCAT  CGGCAAAATG  GGGGGAGCTA
GAAACGGCTC  GGCAGGAGAA  AGCTATCTCA
GGGGTAACCA  GATTGAAGCT  CCGCTTGGCG
TCGCTTGCCC CCGTCCTGAA AAAACACGTC AA-
CAGCGATT TGTTCCGAAA AGCCTCTGAT CGAA-
TAGAGT   CGATAGAGTA   TACGTTGGAA
ACCTTGCGTA TAATGAAAAC TTTCTTCGAT GG-
TACCTCTC TTGAGGGAGA TACTTCCGTA CGT-
GACTCGT   ATATGGCGGG   CGTAGTAGAT
GGAATGGTTC GAGCGAATCC GGATGTGAAG
ATAATTCTGC  TGGCGCACAA  CAATCATCTA
CAAAAAACTC CAGTCTCCTT TTCAGGCGAG CT-
TACGGCTG TTCCCATGGG GCAGCACCTC GCA-
GAGAGGG  TGAATTACCG  TGCGATTGCA  TT-
CACCCATC TTGGACCCAC CGTGCCGGAA ATG-
CATTTCC   CATCGCCAAA   AAGTCCTCTT
GGATTCTCTG  TTGTGACCAC  GCCTGCCGAT
GCAATCCGTG  AGGATAGTAT  GGAACAGTAT
GTCATCGACG  CCTGTGGTAC  GGAGAATTCA
TGTCTGACAT  TGACAGATGC  CCCCATGGAA
GCAAAGCGAA  TGCGGTCTCA  AAGCGCCTCT
GTAGAAACGA  AATTGAGCGA  GGCATTTGAT
GCCATCGTCT  GTGTTACAAG  CGCCGGCAAG
GACAGCCTGG TTGCCCTA
```

2. Procédé selon la revendication 1, caractérisé en ce que la sonde est un ADN recombinant contenant tout ou partie du gène ereA .

3. En tant que produit nouveau, une séquence nucléotidique contenant au plus 1500 paires de bases, telle qu'isolée à partir du plasmide pIP 1100, cette séquence nucléotidique contenant tout ou partie du gène ereA lui-même.

4. Vecteur recombinant modifié par une insertion constituée par la séquence nucléotidique de la revendication 3.

FIG.1

```
CCGGGTTATT  GGCGAACGAA  GTATGACGAT  TACAGCAATA  AACGCAAAGG   50
TAAAAAAATG  ACATTGGAGA  ACGACCAGAA  CACTTTTACA  GCCTCAAAAG  100
CTGGACTTCA  ATGAGTTTGA  GATTCTTACT  TCCGTAATTG  AGGGCGCCCG  150
AATTGTCGGC  ATTGGCGAGG  GCGCTCATTT  TGTCGCGGAG  TTTTCACTGG  200
CTAGAGCTAG  TCTTATCCGC  TATTTGGTCG  AAAGGCATGA  GTTTAAATGC  250
GATTGGTTTG  GAAATGTGGG  GCGATTCAGG  CATCCCGGTT  ATCTGAATGG  300
CTCAACTCAA  CAGCCGGTGC  TCATGAACTT  GAGCGATTTT  CGGATACCCT  350
GACCTTTTCT  GTGTATGGCT  CAGTGCTGAT  CTGGCTGAAA  TCATATCTCC  400
GCGAATCAGG  AAGAAAACTG  CAGTTAGTCG  GAATCGCCTT  ACCCAACACC  450
CTGAACCCAA  GGGACGACCT  AGCGCAATTG  GCCGAAATTA  TCCAGCTCAT  500
CGATCACCTC  ATGAAACCGC  ACGTTGATAT  GTTGACTCAC  TTGTTGGCGT  550
CCATTGATGG  CCAGTCGGCG  GTTATTTCAT  CGGCAAAATG  GGGGGAGCTA  600
GAAACGGCTC  GGCAGGAGAA  AGCTATCTCA  GGGGTAACCA  GATTGAAGCT  650
CCGCTTGGCG  TCGCTTGCCC  CCGTCCTGAA  AAAACACGTC  AACAGCGATT  700
TGTTCCGAAA  AGCCTCTGAT  CGAATAGAGT  CGATAGAGTA  TACGTTGGAA  750
ACCTTGCGTA  TAATGAAAAC  TTTCTTCGAT  GGTACCTCTC  TTGAGGGAGA  800
TACTTCCGTA  CGTGACTCGT  ATATGGCGGG  CGTAGTAGAT  GGAATGGTTC  850
GAGCGAATCC  GGATGTGAAG  ATAATTCTGC  TGGCGCACAA  CAATCATCTA  900
CAAAAAACTC  CAGTCTCCTT  TTCAGGCGAG  CTTACGGCTG  TTCCCATGGG  950
GCAGCACCTC  GCAGAGAGGG  TGAATTACCG  TGCGATTGCA  TTCACCCATC 1000
TTGGACCCAC  CGTGCCGGAA  ATGCATTTCC  CATCGCCAAA  AAGTCCTCTT 1050
GGATTCTCTG  TTGTGACCAC  GCCTGCCGAT  GCAATCCGTG  AGGATAGTAT 1100
GGAACAGTAT  GTCATCGACG  CCTGTGGTAC  GGAGAATTCA  TGTCTGACAT 1150
TGACAGATGC  CCCCATGGAA  GCAAAGCGAA  TGCGGTCTCA  AAGCGCCTCT 1200
GTAGAAACGA  AATTGAGCGA  GGCATTTGAT  GCCATCGTCT  GTGTTACAAG 1250
CGCCGGCAAG  GACAGCCTGG  TTGCCCTATA  GGAAACCGGA  AATGAAAATG 1300
AGGGAGCATA  ACCTGCGAAT  CCACCGGACG  GTTTTCAACC  GCCGGTGATC 1350
AGTTGGGTGC  ACGAGTGGGT  TACATCGAAC  TGGATC
```

## FIG.2

FIG.3

FIG.4

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl 4) |
|---|---|---|---|
| A | BIOLOGICAL ABSTRACTS, vol. 81, no. 1, janvier 1986, no. 1535, Philadelphia, US; A. ANDREMONT et al.: "Plasmid-mediated susceptibility to intestinal microbial antagonisms in Escherichia-coli", & INFECTION AND IMMUNITY (US) 1985, vol. 49, no. 3, pages 751-755 * En entier * | 1-4 | C 12 Q 1/68<br>C 07 H 21/04<br>C 12 N 15/00<br>C 12 N 9/16 |
| A | CHEMICAL ABSTRACTS, vol. 89, 1978, page 187, résumé no. 219592h, Columbus, Ohio, US; J.G. SUTCLIFFE: "Nucleotide sequence of the ampicillin resistance gene of Escherichia coli plasmid pBR322", & PROC. NATL. ACAD. SCI. U.S.A. 1978, 75(8), 3737-41 | | |
| A | CHEMICAL ABSTRACTS, vol. 102, no. 15, 15 avril 1985, page 332, résumé no. 128638f, Columbus, Ohio, US; P. BARTHELEMY et al.: "Enzymic hydrolysis of erythromycin by a strain of Escherichia coli. A new mechanism of resistance", & J. ANTIBIOT. 1984, 37(12), 1692-6 | | |

---                     -/-

**DOMAINES TECHNIQUES RECHERCHES (Int. Cl 4)**

C 12 Q
C 12 N

Le present rapport de recherche a ete etabli pour toutes les revendications

| Lieu de la recherche | Date d'achèvement de la recherche | Examinateur |
|---|---|---|
| LA HAYE | 21-10-1986 | OSBORNE H.H. |

| DOCUMENTS CONSIDERES COMME PERTINENTS | | Page 2 | |
|---|---|---|---|
| Catégorie | Citation du document avec indication. en cas de besoin. des parties pertinentes | Revendication concernee | CLASSEMENT DE LA DEMANDE (Int. Cl 4) |
| T | GENE, vol. 35, no. 3, septembre-décembre 1985, pages 271-277, Elsevier, Amsterdam, NL; H. OUNISSI et al.: "Nucleotide sequence of the gene ereA encoding the erythromycin esterase in Escherichia coli" * En entier * ----- | 1-4 | |
| | | | DOMAINES TECHNIQUES RECHERCHES (Int. Cl.4) |

Le présent rapport de recherche a été établi pour toutes les revendications

| Lieu de la recherche LA HAYE | Date d'achevement de la recherche 21-10-1986 | Examinateur OSBORNE H.H. |
|---|---|---|